Europäisches Patentamt

European Patent Office

Office européen des brevets

(11) Publication number: **0 254 439**
**A1**

## EUROPEAN PATENT APPLICATION

(21) Application number: **87305824.2**

(22) Date of filing: **01.07.87**

(51) Int. Cl.4: **A61F 2/64**

(30) Priority: **18.07.86 GB 8617584**

(43) Date of publication of application:
**27.01.88 Bulletin 88/04**

(84) Designated Contracting States:
**DE FR**

(71) Applicant: **J.E. HANGER & COMPANY LIMITED**
**Roehampton Lane**
**Roehampton London SW15 5PL(GB)**

(72) Inventor: **Cooper, John Edwin**
**St. Gallen**
**The Ballands North Leatherhead KT22**
**9HU(GB)**

(74) Representative: **Cole, Paul Gilbert et al**
**HUGHES CLARK & CO. 63 Lincoln's Inn**
**Fields**
**London WC2A 3JU(GB)**

(54) Knee prosthesis.

(57) A knee prosthesis comprises upper and lower hinge members (10, 12), a knee pivot (14) interconnecting the hinge members (10, 12) at a posterior location and means at an anterior location for locking the knee in an unflexed position. The locking means comprises a body (16) supported for rotation in the upper hinge member (10) about an axis generally parallel to the knee pivot (14) and carrying latch means (54) rotatable into engagement with catch means (42) on the anterior of the lower hinge member (12). Desirably the geometry of the latch means (16, 54), catch means (42) and knee pivot (14) is such that the lock pulls tight under load. The knee may be provided with control means. For this purpose J-links (88) extend from a shin tube (80) depending from the lower hinge member (12) to a location (90) on the upper hinge member (10) that crosses above the knee pivot (14) from the anterior to the posterior side thereof as the knee flexes whereby a load in the links (88) urges the knee towards extension at small angles of flexion, has only a small effect at angles of flexion of about 90° and urges the knee towards its fully flexed position at high angles of flexion. For extension assist the links (88) are rigid and interconnect the upper hinge member (10) with a sliding collar (96) on the shin tube (80) so that the collar (96) oscillates along the shin tube (80) as the knee flexes and extends, at least one control means (106) being operatively connected to the sliding collar for altering the natural motion of the upper and lower hinge members (10, 12) as the leg flexes and extends.

FIG.2.

FIG.12.

1a.

## KNEE PROSTHESIS

This invention relates to a knee prosthesis of an artifical leg of the so-called uniaxial type.

It is an object of the invention to provide a uniaxial knee prosthesis suitable for use as part of a modular endoskeletal limb that is compact and of light weight and inexpensive construction but that nevertheless can be made to comply with strength and fatigue requirements that enable it to be fitted to a full range of patients.

It is a further object of the invention to provide a uniaxial knee as aforesaid provided with extention lock means that is not forced out of engagement under load.

It is a further object of the invention to provide extension assist means and swing phase control means for use in association with a knee prosthesis as aforesaid.

In one aspect the invention provides a knee prosthesis comprising upper and lower hinge members, a knee pivot interconnecting the hinge members at a posterior location and means at an anterior location for locking the knee in an unflexed position, wherein said locking means comprises a body supported for rotation in the upper hinge member about an axis generally parallel to the knee pivot and carrying latch means rotatable into engagement with catch means on the anterior of the lower hinge member.

The invention further provides a knee prosthesis comprising upper and lower hinge members, and a knee pivot interconnecting the hinge members at a posterior location wherein links extend from a shin tube depending from the lower hinge member to a location on the upper hinge member that crosses above the knee pivot from the anterior to the posterior side thereof as the knee flexes whereby a load in the links urges the knee towards extension at small angles of flexion, has only a small effect at angles of flexion of about 90° and urges the knee towards its fully flexed position at high angles of flexion.

The knee prosthesis preferably has rigid links of J-profile, their upper ends being pivoted at an anterior location to the upper hinge member and their lower ends facing in a posterior direction and being pivoted to a collar that is slideable along the shin tube, resilient means biasing the collar away from the hinge members. The shin tube preferably carries a sleeve defining a bearing surface. The sleeve on which the collar slides may be of plastics material whilst the shin tube may be of aluminium or carbon fibre. For swing-phase control the collar has depending spigot means having a female conical inner surface cooperating with segmented collet means on the shaft having a male conical outer surface that mates with the female surface of the spigot means, and second resilient means biases the collet means towards the spigot means whereby the collet means provides a frictional resistance to relative movement of the upper and lower hinge members.

In a yet further aspect, the invention provides a knee prosthesis comprising upper and lower hinge members and a knee pivot interconnecting the hinge members at a posterior location, wherein rigid links interconnect the upper hinge member with a sliding collar on a shin tube depending from the lower hinge member so that the collar oscillates along the shin tube as the knee flexes and extends, at least one control means being operatively connected to the sliding collar for altering the natural motion of the upper and lower hinge members as the leg flexes and extends.

Other preferred features of the invention are described in the appended claims to which attention is hereby directed.

An embodiment of the invention will now be described by way of example only, with reference to the accompanying drawings, in which:-

Figures 1 and 2 are front and side elevations respectively of an artificial knee in its unflexed position;

Figures 3 and 4 are side elevations of the knee of Figure 1 at angles of flexion of 90° and 135° respectively;

Figures 5 and 6 are a side elevation and an underneath plant respectively of an upper hinge member forming part of the knee;

Figure 7 is a top plan view of a lower hinge member forming part of the knee;

Figures 8, 9 and 10 are side, sectional and end views respectively of a locking member forming part of the knee;

Figures 11, 12 and 13 are front, side and sectional views respectively of the knee fitted to a shin tube and further provided with extension assist and frictional damping means;

Figure 14 is a diagrammatic side view of the locking parts of the joint showing the geometrical condition for an effective knee lock; and

Figures 15 and 16 are side and front views of a body and a latch bar respectively of a two part latch according to a second embodiment of the invention.

In the drawings, a knee prosthesis comprises an upper hinge member 10 and a lower hinge member 12 connected together at a posterior position by means of a transversely directed pivot pin 14 defining a knee centre and lockable in an unflexed position be means of a rotatable latch 16 located in an anterior position.

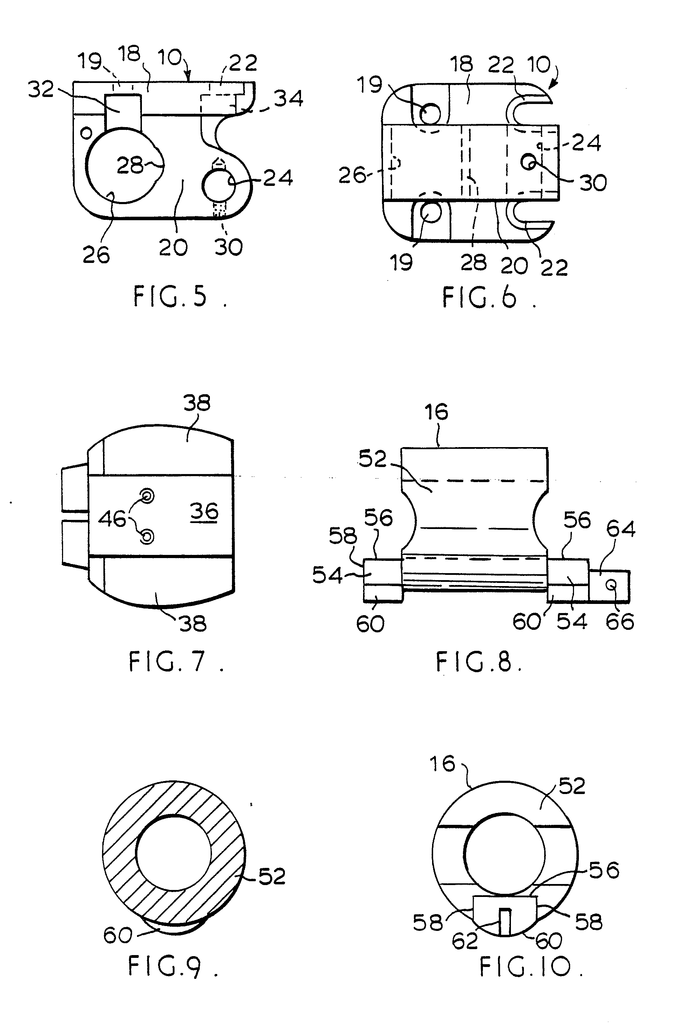

As shown in Figures 5 and 6, the top hinge 10 is of generally T-profile when viewed from the front with a top plate 18 having anterior through-holes 19 and slotted posterior through-holes 22 permitting upper parts of a limb prosthesis to be mounted thereon. A web 20 depends from the plate 18. It is formed with a posterior through-hole 24 in which the pin 14 fits and and an anterior through-hole 26 in which a body of the rotatable latch 16 fits and which is formed with a posteriorly-directed lobe 28 permitting the latch 16 to be inserted as described below during assembly of the knee. A threaded bore 30 from the underside of web 20 intersects the through-hole 24 and accommodates a grub-screw that holds the pin 14 so that the pin 14 moves with the upper hinge member 10. Portions of the web 20 and plate 18 about the holes 19, 22 are removed as at 32, 34 to give allen key access from underneath to fixing bolts in the holes 19, 22.

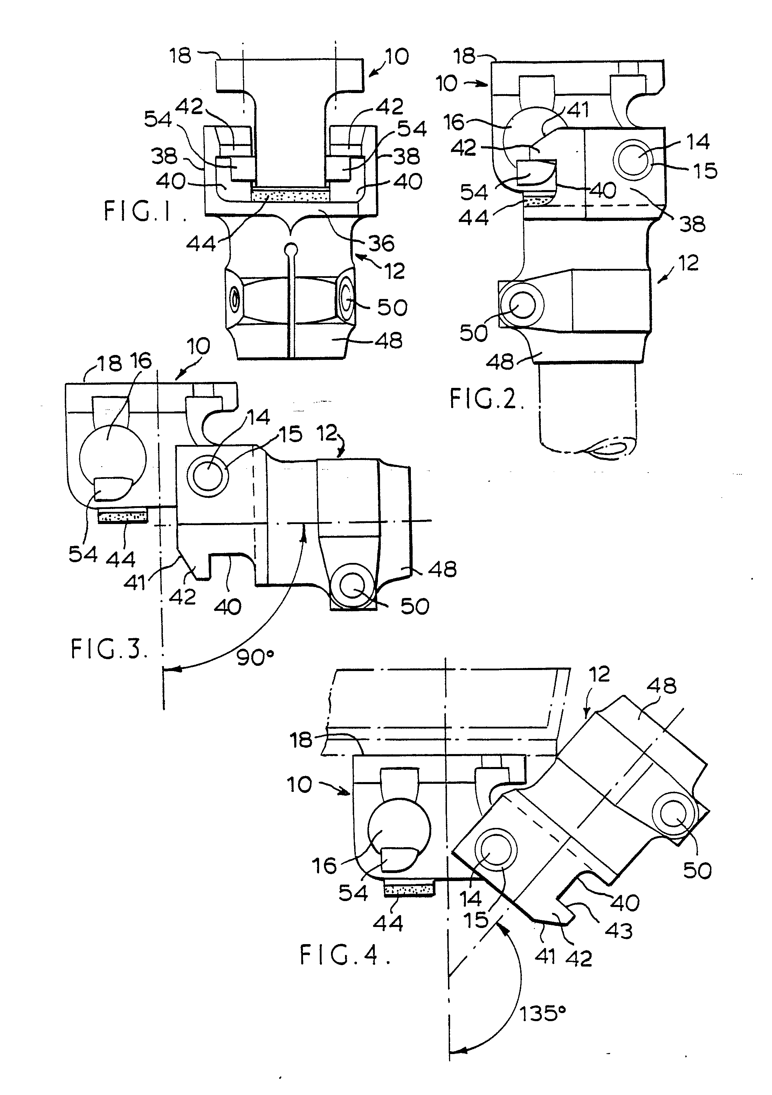

Referring to Figures 1, 2 and 7, the bottom hinge 12 has a head of generally U-shape defined by a baseplate 36 and cheek plates 38 that are apertured to receive the pin 14. Bearing inserts 15 in the cheek plates 38 rotatably support the ends of pin 14 - the arrangement where pin 14 is fixed for rotation in the upper hinge member 10 and is carried in bearings in the cheek plates of the lower hinge member reducing bearing load. Transverse curvilinear recesses 40 formed in an anterior edge of each cheek plate 38 lead to catch-defining noses 42. A buffer 44 of hard elastomeric material may be held on the web 20 of the upper hinge member as in Figures 3 and 4 but is preferably held on the baseplate 36 by screws that are received in fixing holes 46 (Figures 7). A slotted shin tube socket 48 of aluminium alloy or of carbon fibre reinforced resin depends from the baseplate 44 and may be clamped onto a shin tube by means of a pinch bolt 50.

As shown in Figures 8 to 10, one embodiment of the latch 16 has a cylindrical or tublar body 52 formed on its end faces with eccentric latch bars 54 each located adjacent the cylindrical outer surface of body 52 with straight top and side faces 56, 58 directed at 90° to one another and with a domed lower face 60 whose profile is matched by the lobe 28 in the top hinge. Accordingly the latch 16 may be fitted to the hinge member 10 at a rotational position such that latch bars 54 coincide with the lobe 28 and is then rotated so that the bars 54 face downwardly (locking position) or toward the anterior (release position) in both of which positions the web 20 of the top plate fits between the bars 54 so that the latch 16 cannot be removed. One of the latch bars 54 is formed with a hole 62 extending upwards from lower face 60 for reception of one end of a spring (not shown) that urges the latch bars 54 towards the posterior direction to effect locking of the knee. The other latch bar 54 is formed with a stub handle 64 formed with a hole 66 that receives an end of a release cable (not shown) by which the latch bars 54 may be pulled in a posterior direction.

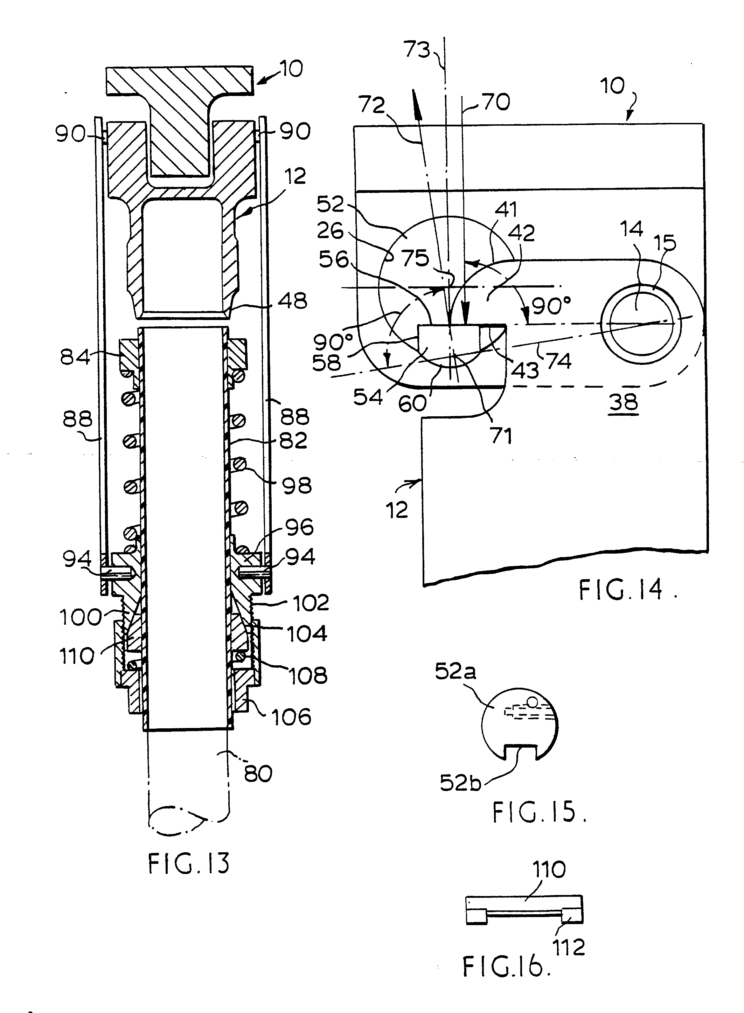

Operation of the knee lock may be understood by reference to Figure 14. As the knee is unflexed the latch bars 54 encounter the top edges 41 of the cheek plates 38 by which they are cammed in an anterior direction against the resistance of the spring, the body 52 rotating anticlockwise as viewed in Figure 14 in the through-hole 26. When the faces 56 of the latch bars 54 pass the lower faces 43 of the noses 42 the latch bars 54 snap into the locking positions shown. It is desirable that the geometry of the latch 16, catch noses 42 and knee pivot 14 are such that the lock tends to pull tight under load. In order for this to happen:

(a) the face 43 should be directed towards the centre of the knee pivot 14 or should be directed slightly downwardly;

(b) the line 70 of pressure of the face 56 on the face 43 should be directed upwardly or outwardly but not inwardly - i.e. the angle between the line of pressure 70 and a line joining abutting faces 43, 56 to the centre of the knee pivot 14 should not be less than 90°;

(c) In order for this to happen, the location 71 where tangent 74 touches the cylindrical surface of body 52 should lie in or to the same side of lock centre line 73 as the knee pivot 14. The lock centre line 73 is defined by passage of a medial/lateral plane through the axis 75 of the body 52, a line of pressure 72 between the web 20 and the body 52 of the latch 16 should be directed upwardly or outwardly but not inwardly. The line of pressure 72 is given by a tangent 74 to the cylindrical surface of body 52 that passes through the centre of pin 14; and

(d) the profile of latch noses 42 and latch bars 54 should be such that the body 52 can rotate counter-clockwise as viewed in Figure 14 freeing bars 54 from nose 52 and permitting the joint to flex. In Figure 14 the noses 42 do not extend beyond the lock centre line 73 and the face 43 is flat. If the nose 42 extends beyond centre line 73 (which is not preferred) then the face 43 is curvilinear with an outer radiused region being profiled so as not to be found by the latch bars 54 as they are moved into the locking position.

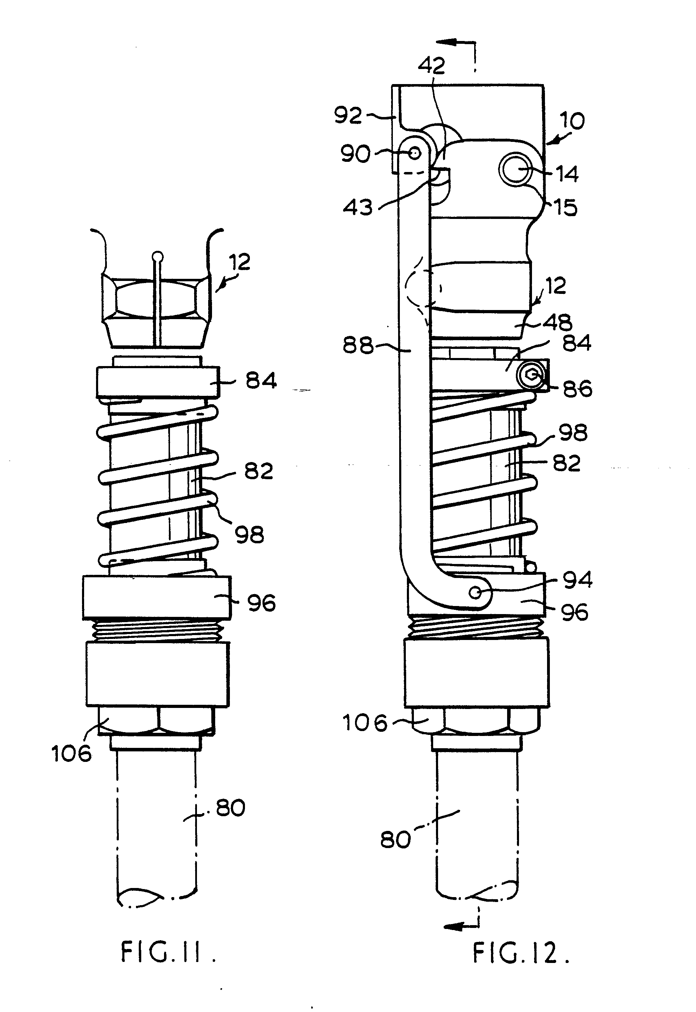

As shown in Figures 11 to 13, the joint may be provided with optional extension assist and swing phase friction damping mechanisms.

A shin tube 80 fitting into the socket 48 carries a sleeve 82 which is preferably of plastics material and a collar 84 clamped in position axially of tube 80 by means of pinch bolt 86. Generally J-shaped links 88 are each pivoted at one end at pivot pin 90 to a carrier 92 attached to the front of the upper hinge member 10 and are each pivoted at their other end at pin 94 to a sliding collar 96 that moves over the sleeve 82 below the fixed collar 84. J-shaped links are the most convenient for this purpose, but C-shaped or lazy S-shaped links can also be used. The reason for use of J-links or other shaped links of rigid material is to avoid interference with shin components at a fully flexed position of the knee. A coil spring 98 in compression between collars 84 and 96 urges collar 96 downwardly relative to collar 84. At small angles of knee flexion further flexion of the knee raised collar 96 on shin tube 80 against the resistance of spring 98, thereby providing an extension assist function. At about 90° of knee flexion the pivots 90, 14 and 94 are aligned and the spring 94 produces no net moment about the knee axis 14. As knee flexion is further increased the upper pivot 90 of link 88 passes to the posterior side of a line joining lower pivot 94 of the link 88 to the centre of pivot pin 14. Further flexion of the knee causes collar 96 to fall on shin tube 80 assisted by spring 98 which urges the knee towards the fully flexed position of Figure 4. The action of spring 98 is therefore to provide extension assist at small degrees of knee flexion typically up to about 55°, to provide no effective moment at angles of about 90° corresponding to a sitting attitude and to urge the knee towards its fully flexed position at higher flexion angles, assisting in overcoming the resistance to bending of a cosmesis that fits over the knee in the finished limb.

For swing phase friction damping the sliding collar 96 has a depending spigot 100 having a threaded outer surface 102 and a tapered inner surface 104. A retaining collar 106 is a screw fit onto the surface 104 and holds a coil spring 108 in compression against a divided segmented collet 110 having a tapered top surface that mates with the surface 104 of the spigot. The spring 108 urges the collet 110 upwardly and into the taper of surface 104 whereby it presses against the sleeve 102 to provide a frictional resistance to movement of the collar 96. The value of that resistance is dependent upon the compression of spring 108 which is set by rotating the collar 106. The effect of the frictional resistance depends on the moment applied through links 88 about the pin 14, and that moment is significant at angles of up to about 55°

characteristic of the swing phase of walking, falls to a low value at angles close to 90° corresponding to a sitting attitude and rises again. The collet 110 is suitably of hard anodised aluminium and travels over a sleeve 102 of glass-filled nylon, though the material could be reversed or other dissimilar materials could be used.

It will be appreciated that modifications may be made to the embodiment described above without departing from the invention. For example, if provision for extension assist is required without damping, straps of elastomeric material can simply be stretched between fixing points corresponding to pivots 90, 94 without the need for links 88, collars 84, 96 and spring 98. In Figures 16 and 17 a one-piece body and latch bars is replaced with a body 52a formed with a transverse recess and a latch member 110 in the form of a bar having latch bars 112 at its ends. In assembly the member 110 is inserted into the anterior through-hole, after which the body 52a inserted with the member 110 received in the recess 52a. A simple through-hole without a lobe is all that is required.

## Claims

1. A knee prosthesis comprising upper and lower hinge members (10, 12) a knee pivot (14) interconnecting the hinge members (10, 12) at a posterior location and means at an anterior location for locking the knee in an unflexed position, characterised in that said locking means comprises a body (16) supported for rotation in the upper hinge member (10) about an axis generally parallel to the knee pivot (14) and carrying latch means (54) rotatable into engagement with catch means (42) on the anterior of the lower hinge member.

2. A prosthesis according to Claim 1, wherein the upper hinge member (10) is generally of T-front profile with a web portion thereof (20) fitting between cheek plates (38) of the lower hinge member (12), the knee pivot (14) is attached for rotation with the upper hinge member (10) and is received in bearings (15) in the cheek plates (38) of the lower hinge member (12), the body (52) of said locking means (16) has eccentrically located latch bars (54) extending from its opposed ends that are engageable with noses (42) in the anterior faces of the cheek plates (38) and resilient means operatively connected to the body (52) urges the latch bars (54) into snap engagement with the noses (42) of the cheek plates (38).

3. A prosthesis according to Claim 2, wherein when the latch bars (54) are located adjacent the side surface of the body and when viewed in profile extend beyond the side surface of the body, and wherein an aperture (26) in the upper hinge member (10) is formed with a lobe (28) of profile corresponding to that of the latch bars (54), 60) so that the body (52) can be inserted into the aperture in an inoperative attitude and rotated to operative attitudes where it is captive in the upper hinge member (10).

4. A prosthesis according to Claim 2, wherein one of the latch bars (54) is extended to define a stub handle (64) to which a release cable is attachable, and the other latch bar (54) is operatively connected to one end of the resilient means.

5. A prosthesis according to any preceding claim, wherein the pressures exerted by the latch means (54) on the catch means (42) and by the upper knee member on the body under a load urging the knee to flex act along or outwardly of a line (73) parallel to the knee axis whereby the latch is not forced out of engagement as the load increases.

6. A prosthesis according to Claim 5, wherein undersurface means (43) of the catch means (42) engaged by the latch means (54) is directed so that the knee pivot (14) is in alignment therewith, and the rotatable body (52) is located in the upper hinge member (10) so that a tangent (74) to its cylindrical surface that passes through the knee axis (14) touches the cylindrical surface at a position (71) that coincides with, or is closer to the knee axis (14) than a medial/lateral plane (73) passing through the axis of rotation of the rotatable body (52).

7. A knee prosthesis comprising upper and lower hinge members (10, 12), and a knee pivot (14) interconnecting the hinge members (10, 12) at a posterior location, characterised in that links (88) extend from a shin tube (80) depending from the lower hinge member (12) to a location (90) on the upper hinge member (10) that crosses above the knee pivot (14) from the anterior to the posterior side thereof as the knee flexes whereby a load in the links urges the knee towards extension at small angles of flexion, has only a small effect at angles of flexion of about 90° and urges the knee towards its fully flexed position at high angles of flexion.

8. A knee prosthesis according to Claim 7, wherein either (a) the links are in tension and are straps of elastomeric material fastened between the upper hinge member (10) and the shin tube (80) or (b) the links (88) are rigid and are of J-profile, their upper ends are pivoted at an anterior location (90) to the upper hinge member (10) and their lower ends face in a posterior direction and are pivoted to a collar (96) that is slideable along the shin tube (80), resilient means (98) biasing the collar away from the hinge members to tension the links.

9. A prosthesis according to Claim 8, wherein the shin tube (80) carries a sleeve (82) on which the collar (84) slides, the sleeve (82) being of plastics material and the shin tube (80) being of aluminium or carbon fibre.

10. A prosthesis according to Claim 8 or 9, wherein the collar (96) has depending spigot means (100) having a female conical inner surface (104) cooperating with segmented collet means (110) on the shaft having a male conical outer surface that mates with the female surface (104) of the spigot means (100), and second resilient means (108) biases the collet means (110) towards the spigot means (100) whereby the collet means (110) provides a frictional resistance to relative movement of the upper and lower hinge members (10, 12), the second resilient means being retained by retaining means (106) threadedly engaged with the spigot means (100) so that rotation of the retaining means (106) adjusts the load exerted on the collet means (110) by the second resilient means (108) whereby the friction load on the links (88) during flexion and extension is varied.

11. A knee prosthesis comprising upper and lower hinge members (10, 12) and a knee pivot (14) interconnecting the hinge members (10, 12) at a posterior location, characterised in that rigid links (88) interconnect the upper hinge member (10) with a sliding collar (96) on a shin tube (80) depending from the lower hinge member (12) so that the collar (96) oscillates along the shin tube (80) as the knee flexes and extends, at least one control means (98) being operatively connected to the sliding collar (96) for altering the ntural motion of the upper and lower hinge members (10, 12) as the leg flexes and extends.

12. A prosthesis according to Claim 11, wherein the control means comprises either (a) resilient means (98) biasing the knee prosthesis to an extended position at low angles of flexion, said resilient means (98) being a coil spring on the shin tube (80) and held in compression between the sliding collar (96) and fixed abutment means (84) on the shin tube (80), or (b) collet means (110) slideable over a bearing surface (82) on the shin tube (80) and means (104, 108) for urging the collet means (110) against the shin tube (80) to provide a frictional resistance to relative movement of the upper and lower hinge members (10, 12).

FIG.1.

FIG.2.

FIG.3.

90°

FIG.4.

135°

FIG. 5 .

FIG. 6 .

FIG. 7 .

FIG. 8.

FIG. 9 .

FIG. 10 .

FIG.11.

FIG.12.

FIG.13

FIG.14.

FIG.15.

FIG.16.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-C- 317 320 (GRUNDLER et al.) <br> * figures 1-3 * | 1 | A 61 F 2/64 |
| A | FR-A- 768 306 (JUENIN-HANGER) <br> * figures 1-5 * | 1 | |
| A | FR-A- 517 988 (CHAUMETTE) <br> * figures * | 1 | |
| A | DE-C- 309 817 (HUBER) <br> * figures 1-4 * | 1 | |

TECHNICAL FIELDS SEARCHED (Int. Cl.4)

A 61 F 2/00

The present search report has been drawn up for all claims

| Place of search | Date of completion of the search | Examiner |
|---|---|---|
| BERLIN | 27-10-1987 | KANAL P K |